# FASCICULE DE BREVET EUROPEEN

(11) **EP 3 434 233 B1**
(45) Date de publication et mention de la délivrance du brevet: **09.09.2020**
(21) Numéro de dépôt: 18183951.5
(22) Date de dépôt: 17.07.2018
(51) Int. Cl.: A61F 9/007

(54) **SONDE BICANALICULAIRE AUTOSTABLE**
SELBSTSTEHENDE ZWEIKANALIGE SONDE
FREESTANDING BICANALICULAR PROBE

(30) Priorité: 25.07.2017 FR 1770792
(43) Date de publication de la demande: 30.01.2019
(73) Titulaire: France Chirurgie Instrumentation SAS, 75015 Paris (FR)
(72) Inventeur: FERRON, Abraham, 33200 Bordeaux (FR); JONCKHEERE, Paul, 2100 Anvers (BE)
(74) Mandataire: Eidelsberg, Olivier Nathan

(56) Documents cités:
- FR-A1- 2 735 697
- FR-A1- 2 741 538
- US-A- 5 021 043
- US-A- 6 117 116
- US-A1- 2014 364 790

## Description

La présente invention se rapporte à une sonde bicanaliculaire destinée à être insérée dans le canal lacrymonasal d'un patient.

Classiquement, une sonde bicanaliculaire destinée à être insérée dans le canal lacrymonasal d'un patient comporte un tube central formant sonde réalisé en un matériau compatible avec une insertion dans le corps humain, notamment en silicone, qui est relié à ses deux extrémités opposées à des éléments de terminaison, par exemple des fils de guidage, des mandrins ou analogues, qui permettent l'introduction du tube central en silicone dans le canal lacrymonasal.

Ces dispositifs de l'art antérieur, bien que constituant une solution particulièrement appropriée pour être inséré dans le canal lacrymonasal, restent difficiles à manipuler par le chirurgien pour obtenir un bon positionnement dans le canal lacrymonasal et nécessitent de nouer les brins du tube central en fin de pose.

De US 6 117 116, on connaît une sonde suivant le préambule de la revendication 1. Un manchon est formé au niveau de l'interface entre le tube central et les éléments de terminaison respectifs.

De FR 2 735 697, on connaît en outre une technique alternative compliquée, appelée dacryocystorhinostomie ou DCR, dans laquelle on fait passer une sonde dans la fosse nasale sans passer par le canal lacrymonasal obstrué.

La présente invention vise à surmonter les inconvénients de l'art antérieur en proposant une sonde bicanaliculaire destinée à être insérée dans le canal lacrymonasal d'un patient, telle que définie à la revendication 1.

De préférence, les deux surépaisseurs sont réalisées sous la forme de deux manchons fixés autour de l'élément tubulaire central.

De préférence, les deux surépaisseurs, notamment les deux manchons, sont à une même distance respective de l'extrémité de fixation de l'élément de terminaison du côté opposé à l'autre surépaisseur, notamment manchon.

De préférence, les deux surépaisseurs, notamment les deux manchons, s'étendent le long du tube sur une longueur comprise entre 4mm et 8mm, notamment entre 5mm et 7mm.

De préférence, la distance entre les deux surépaisseurs, notamment les deux manchons, le long du tube, correspond à un segment central et est comprise entre 30mm et 40mm, notamment entre 32mm et 35mm.

Suivant un mode de réalisation préféré de l'invention, les deux manchons sont collés à l'élément tubulaire central.

Suivant un mode de réalisation préféré de l'invention, les deux terminaisons sont constituées de fils de guidage, la liaison au tube de chaque fil de guidage étant réalisée de manière à ne pas présenter d'aspérité faisant saillie latéralement et pouvant taper « lors de l'insertion ».

Suivant un autre mode de réalisation, on peut prévoir un mandrin métallique, notamment d'un diamètre extérieur de 0,4mm et d'une longueur de 60mm, collé à l'intérieur du tube de silicone.

Suivant un perfectionnement, il est prévu un repère à mi-distance entre les deux manchons, pour aider à la pose par le chirurgien.

En prévoyant une telle sonde bicanaliculaire suivant l'invention, le chirurgien peut faire en sorte que les deux manchons, lors de la pose de la sonde, soient disposés dans le sac lacrymal et non pas dans la partie horizontale des canalicules ni dans le canal lacrymo-nasal. La sonde est ainsi plus mobile et libre en position dans le canal en créant moins de traction sur les méats, évitant les phénomènes de « Cheese wiring ». En outre, il n'est plus nécessaire de nouer les brins de silicone en fin de pose, le retrait s'effectue en tirant sur le segment central, et il n'est plus nécessaire d'aller couper de nœud dans le nez.

La stabilité de la sonde est particulièrement bonne, liée à la présence des deux manchons dans le sac. Enfin le repère central aide à positionner la sonde en tirant, lors de la pose, sur les brins des terminaisons de façon à ce que les manchons se trouvent précisément dans le sac.

A titre d'exemple, on décrit maintenant un mode de réalisation préféré de l'invention en se reportant aux dessins, dans lesquels :
- La figure 1: est une vue schématique d'une sonde bicanaliculaire suivant l'invention ;
- La figure 2: est une vue en coupe suivant la ligne A-A de la figure 1.

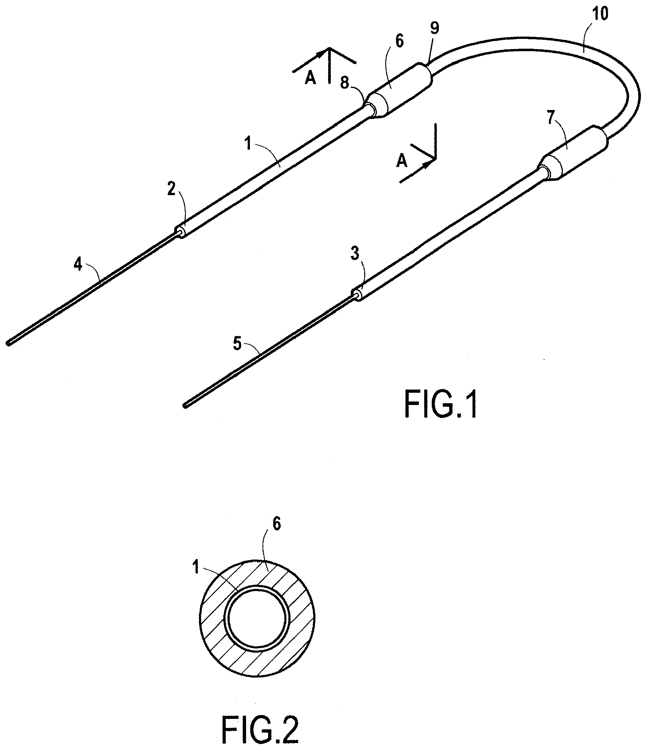

A la figure 1, il est représenté une sonde bicanaliculaire destinée à être insérée dans le canal lacrymo-nasal d'un patient. Cette sonde est constituée d'un tube 1 en silicone qui s'étend entre deux points d'extrémité 2 et 3. Deux éléments de terminaison 4 et 5 sont issus des points d'extrémité 2 et 3 et permettent l'insertion de la sonde. Les éléments 4 et 5 ont une épaisseur plus petite que celle du tube 1.

Ces éléments de terminaison peuvent être des fils de guidage, notamment en PEEK, notamment comme décrit dans le brevet FR-A-2992851 et dont la liaison tube/fil de guidage est chanfreinée pour éviter que toute aspérité puisse taper lors de l'insertion contre la paroi nasale.

Les éléments 4 et 5 peuvent également être des mandrins tels que représentés au document US-A-4380239 et dont la liaison tube/mandrin est également sous forme de chanfrein. Ils peuvent également être des mandrins, notamment de diamètre 0,80mm extérieur et de longueur 80mm, assemblé avec le tube 1 en silicone comme décrit dans le brevet FR-A-2700722. Ils peuvent également être des mandrins métalliques de diamètre extérieur 0,4mm et de longueur 60mm collé à l'intérieur du tube en silicone.

Le tube 1 en silicone a un diamètre de 0,3mm à 0,64mm, la longueur totale du tube en silicone, c'est-à-dire hors terminaisons, étant par exemple de 300mm.

Deux manchons 6 et 7 formant des surépaisseurs du tube font saillie latéralement du tube 1 en silicone. Ils peuvent notamment être fixés au tube par collage, soudage ou analogue. Ils peuvent également être réalisés par moulage ou surmoulage. Ces deux manchons 6 et 7 s'étendent sur une longueur le long du tube 1 comprise entre 4mm et 8mm, notamment entre 5mm et 7mm. Entre les deux manchons 6 et 7, il est défini un segment central du tube 1 en silicone qui a une longueur mesurée le long du tube comprise entre 30mm et 40mm, notamment entre 32mm et 35mm. Au niveau du point médian, c'est-à-dire à mi-distance entre les deux manchons 6 et 7, il est formé un repère 10 central définissant l'exact point milieu pour le chirurgien et lui permettant une pose simplifiée, notamment en s'assurant que les deux manchons 6 et 7 se trouvent bien dans le sac lacrymal.

Le repère 10 central peut être constitué d'un tronçon aminci du tube 1 central. Il peut également être constitué d'un marquage, notamment par tampongraphie avec une encre colorée biocompatible sous la forme d'un point en surface du tube, d'un trait continu ou discontinu sur 360° ou moins en surface autour du tube, d'une portion du tube central remplie d'encre ou de colle silicone (matière translucide), ou toutes autres formes de repères ou marquages analogues.

Les deux manchons ou surépaisseurs s'étendent entre deux points d'extrémité 8, 9 distal et proximal respectifs du manchon 1.

Les deux points 9 proximaux sont à distance l'un de l'autre.

Chaque point 8 distal est à distance du point 2, 3 du tube respectif qui se trouve du côté opposé au point 9 proximal respectif et à partir duquel s'étend l'élément 4, 5 respectif.

## Revendications

1. Sonde bicanaliculaire destinée à être insérée dans canal lacrymonasal d'un patient, comportant un élément (1) central tubulaire réalisé en un matériau compatible avec une insertion dans le corps humain, notamment en silicone, et deux éléments (4, 5) de terminaison fixés chacun à l'élément central de manière à faire saillie de ce dernier à deux extrémités (2, 3) respectives de l'élément tubulaire central, **caractérisée en ce que** deux surépaisseurs (6, 7) à distance l'une de l'autre font saillie de l'élément tubulaire central, les deux surépaisseurs étant chacune à distance des extrémités (2, 3) respectives de l'élément tubulaire central et **en ce que** la distance entre les deux surépaisseurs, notamment les deux manchons, le long du tube, est comprise entre 30 et 40 mm.

2. Sonde suivant la revendication 1, **caractérisée en ce que** les deux surépaisseurs (6, 7) sont réalisées sous la forme de deux manchons fixés autour de l'élément tubulaire central.

3. Sonde suivant la revendication 1 ou 2, **caractérisée en ce que** les deux surépaisseurs, notamment les deux manchons, s'étendent le long du tube sur une longueur comprise entre 5 mm et 7 mm.

4. Sonde suivant l'une des revendications 1 à 3, **caractérisée en ce que** les deux surépaisseurs, notamment les deux manchons, sont à une même distance respective de l'extrémité (2, 3) de l'élément tubulaire central du côté opposé à l'autre surépaisseur, notamment manchon.

5. Sonde suivant l'une des revendications 1 à 4, **caractérisée en ce que** les deux manchons sont collés à l'élément tubulaire central par une couche de colle respective.

6. Sonde suivant l'une des revendications 1 à 5, **caractérisée en ce qu'**il est prévu un repère à mi-distance entre les deux manchons.

## Patentansprüche

1. Zweikanalsonde, die dazu bestimmt ist, in den Tränenkanal eines Patienten eingeführt zu werden, umfassend ein zentrales rohrförmiges Element (1) aus einem zum Einführen in den menschlichen Körper kompatiblen Material, insbesondere Silikon, und zwei Abschlusselemente (4, 5), die jeweils an dem zentralen Element befestigt sind, so dass sie an zwei Enden (2, 3) des zentralen rohrförmigen Elements aus diesem hervorstehen,
**dadurch gekennzeichnet, dass** zwei voneinander beabstandete Verdickungen (6, 7) aus dem zentralen rohrförmigen Element hervorstehen, wobei die beiden Verdickungen jeweils von den jeweiligen Enden (2, 3) des zentralen rohrförmigen Elements beabstandet sind, und dass der Abstand zwischen den beiden Verdickungen, insbesondere den beiden Hülsen, entlang des Rohrs zwischen 30 und 40 mm beträgt.

2. Sonde gemäß Anspruch 1, **dadurch gekennzeichnet, dass** die beiden Verdickungen (6, 7) in Form von zwei Hülsen ausgeführt sind, die um das zentrale rohrförmige Element herum befestigt sind.

3. Sonde gemäß Anspruch 1 oder 2, **dadurch gekennzeichnet, dass** die beiden Verdickungen, insbesondere die beiden Hülsen, sich entlang des Rohrs über eine Länge von zwischen 5 mm und 7 mm erstrecken.

4. Sonde gemäß einem der Ansprüche 1 bis 3, **dadurch gekennzeichnet, dass** die beiden Verdickungen, insbesondere die beiden Hülsen, jeweils auf der der anderen Verdickung, insbesondere Hülse, gegenüberliegenden Seite den gleichen Abstand zum Ende (2, 3) des zentralen rohrförmigen Elements aufweisen.

5. Sonde gemäß einem der Ansprüche 1 bis 4, **dadurch gekennzeichnet, dass** die beiden Hülsen durch jeweils eine Klebstoffschicht mit dem zentralen rohrförmigen Element verklebt sind.

6. Sonde gemäß einem der Ansprüche 1 bis 5, **dadurch gekennzeichnet, dass** in der Mitte des Abstands zwischen den beiden Hülsen eine Markierung vorgesehen ist.

## Claims

1. A bicanalicular probe intended to be inserted into the nasolacrimal duct of a patient, comprising a central tubular part (1) made from a material compatible with insertion into the human body, preferably silicone, and two termination parts (4, 5) each fixed to the central part so that they project from two respective ends (2, 3) of the central tubular part, **characterised in that** two thickened parts (6, 7) at a distance from one another form projections from the central tubular part, the two thickened parts each being at a distance from the respective ends (2, 3) of the central tubular part, and **in that** the distance along the tube between the two thickened parts, notably the two tubes, is comprised between 30 mm and 40 mm.

2. A probe according to claim 1, **characterised in that** the two thickened parts (6, 7) are in the form of two tubes fixed around the central tubular part.

3. A probe according to one of claims 1 or 2, **characterised in that** the two thickened parts, notably the two tubes, extend along the tube for a length of between 5 mm and 7 mm.

4. A probe according to one of claims 1 to 3, **characterised in that** the two thickened parts, notably the two tubes, are at the same respective distance from the ends (2, 3) of the central tubular part on the side opposite to the other thickened part, notably the tube.

5. A probe according to one of claims 1 to 4, **characterised in that** the two tubes are each bonded to the central tubular part by a respective layer of adhesive.

6. A probe according to one of claims 1 to 5, **characterised in that** there is provided a mark midway between the two tubes.
